# EUROPÄISCHE PATENTSCHRIFT

(11) **EP 0 917 474 B1**
(45) Veröffentlichungstag und Bekanntmachung des Hinweises auf die Patenterteilung: **18.12.2002**
(21) Anmeldenummer: 97929292.7
(22) Anmeldetag: 25.06.1997
(51) Int. Cl.: A61K 49/00, A61K 47/48, A61K 49/04

(54) **PSEUDOPOLYROTAXANE**
PSEUDOPOLYROTAXANES
PSEUDOPOLYROTAXANES

(30) Priorität: 09.07.1996 DE 19629494
(43) Veröffentlichungstag der Anmeldung: 26.05.1999
(73) Patentinhaber: SCHERING AKTIENGESELLSCHAFT, 13353 Berlin (DE)
(72) Erfinder: PLATZEK, Johannes, Dr., D-12621 Berlin (DE); SCHMITT-WILLICH, Heribert, Dr., D-12161 Berlin (DE)
(86) Internationale Anmeldenummer: EP9703344
(87) Internationale Veröffentlichungsnummer: WO98001163

(56) Entgegenhaltungen:
- EP-A- 0 766 968
- US-A- 4 986 980
- US-A- 5 336 762
- AKIRA HARADA ET AL: "SYNTHESIS OF A TUBULAR POLYMER FROM THREADED CYCLODEXTRINS" NATURE, Bd. 364, Nr. 6437, 5.August 1993, Seiten 516-518, XP000371695
- RAYMO F M ET AL: "POLYROTAXANES AND PSEUDOPOLYROTAXANES" TRENDS IN POLYMER SCIENCE, Bd. 4, Nr. 7, 1.Juli 1996, Seiten 208-211, XP000591869
- CARDENAS D.J. ET AL: "Construction of interlocking and threaded rings using two different transition metals as a templating and connecting centers: Catenanes and rotaxanes incorporating Ru(terpy)2-units in their framework" JOURNAL OF THE AMERICAN CHEMICAL SOCIETY, 1997, 119/11 (2656-2664), USA, XP002054618

## Beschreibung

Die Erfindung betrifft den in den Patentansprüchen gekennzeichneten Gegenstand, das heißt neue Pseudopolyrotaxane, diese Verbindungen enthaltende Mittel, die Verwendung dieser Verbindungen in der Diagnostik sowie Verfahren zur Herstellung dieser Verbindungen und Mittel.

Pseudopolyrotaxane sind Verbindungen, in denen mehrere ringförmige Moleküle auf einem geeigneten Polymerrückgrat aufgefädelt sind. Solche hochmolekularen Molekülgebilde sind u.a.beschrieben von A. Harada et al., J. Am. Chem., Soc. 1994, 116, 3192-96 und G. Wenz et al., Angew. Chem. 104., 201-204 (1992).

Die zur Zeit klinisch eingesetzten Kontrastmittel für die modernen bildgebenden Verfahren Kernspintomographie (MRI) und Computertomographie (CT) [Magnevist ®, Pro Hance ®, Ultravist® und Omniscan ®] verteilen sich im gesamten extrazelluiären Raum des Körpers (Intravasalraum und Interstitium). Dieser Verteilungsraum umfaßt etwa 20 % des Körpervolumens.

Extrazelluläre MRI-Kontrastmittel sind klinisch zuerst erfolgreich bei der Diagnostik von zerebralen und spinalen Krankheitsprozessen eingesetzt worden, da sich hier eine ganz besondere Situation hinsichtlich des regionalen Verteilungsraumes ergibt. Im Gehirn und im Rückenmark können extrazelluläre Kontrastmittel im gesunden Gewebe aufgrund der Blut-Hirn-Schranke nicht den Intravasalraum veriassen. Bei krankhaften Prozessen mit Störung der Blut-Hirn-Schranke (z.B. maligne Tumoren, Entzündungen, demyelinisierende Erkrankungen etc.) entstehen innerhalb des Hirns dann Regionen mit erhöhter Blutgefäß-Durchlässigkeit (Permeabilität) für diese extrazellulären Kontrastmittel (Schmiedl et al., MRI of blood-brain barrier permeability in astrocytic gliomas: application of small and large molecular weight contrast media, Magn. Reson. Med. 22: 288, 1991). Durch das Ausnutzen dieser Störung der Gefäßpermeabilität kann erkranktes Gewebe mit hohem Kontrast gegenüber dem gesunden Gewebe erkannt werden.

Außerhalb des Gehirns und des Rückenmarkes gibt es allerdings eine solche Permeabilitätsbarriere für die oben genannten Kontrastmittel nicht (Canty et al., First-pass entry of nonionic contrast agent into the myocardial extravascular space. Effects on radiographic estimate of transit time and blood volume. Circulation 84: 2071, 1991). Damit ist die Anreicherung des Kontrastmittels nicht mehr abhängig von der Gefäßpermeabilität, sondern nur noch von der Größe des extrazellulären Raumes im entsprechenden Gewebe. Eine Abgrenzung der Gefäße gegenüber dem umliegenden interstitiellen Raum bei Anwendung dieser Kontrastmittel ist nicht möglich.

Besonders für die Darstellung von Gefäßen wäre ein Kontrastmittel wünschenswert, das sich ausschließlich im vasalen Raum (Gefäßraum) verteilt. Ein solches blood-pool-agent soll es ermöglichen, mit Hilfe der Kernspintomographie gut durchblutetes von schlecht durchblutetem Gewebe abzugrenzen und somit eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Bisher müssen sich die meisten der Patienten, bei denen Verdacht auf eine kardiovaskuläre Erkrankung besteht (diese Erkrankung ist die häufigste Todesursache in den westlichen Industrieländern), invasiven diagnostischen Untersuchungen unterziehen.
Es besteht daher ein Bedarf an NMR- und Röntgenkontrastmitteln, die den vasalen Raum markieren können (blood-pool-agent). Diese Verbindungen sollen sich durch eine gute Verträglichkeit und durch eine hohe Wirksamkeit (hohe Steigerung der Signalintensität bei MRI) auszeichnen.

Der Ansatz, zumindest einen Teil dieser Probleme durch Verwendung von Komplexen, die an Makro- oder Biomoleküle gebunden sind, zu lösen, war bisher nur sehr begrenzt erfolgreich.

So ist beispielsweise die Anzahl paramagnetischer Zentren in den Komplexen, die in den Europäischen Patentanmeldungen Nr. 0 088 695 und Nr. 0 150 844 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromoleküle können generell als Kontrastmittel für die Angiographie geeignet sein. Albumin-GdDTPA (Radiology 1987; 162: 205) z.B. zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.
Das Makromolekül Polylysin-GdDTPA (Europäische Patentanmeldung, Publikations-Nr. 0 233 619) erwies sich ebenfalls geeignet als blood-pool-agent. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthese-bedingt kann Polylysin-GdDTPA aber auch Makromoleküle enthalten, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (Europäische Patentanmeldung, Publikations-Nr. 0 326 226).
Der Nachteil dieser Verbindungen liegt darin, daß diese in der Regel nur ca. 5 % des signalverstärkenden paramagnetischen Kations tragen.

Es bestand daher die Aufgabe, neue diagnostische Mittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Die erfindungsgemäßen Pseudopolyrotaxane lassen sich durch die allgemeine Formel I beschreiben 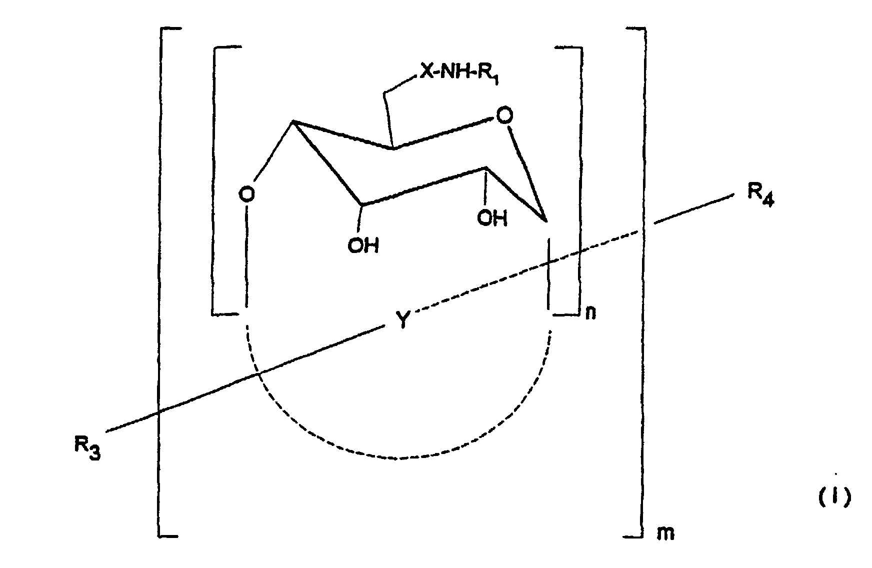 worin
n die Zahlen 6,7 oder 8,
m die Zahlen 2 bis 50,
X eine direkte Bindung oder den Rest -O-CO-CH(CH₃)-
R₁ die kontrastgebenden Reste 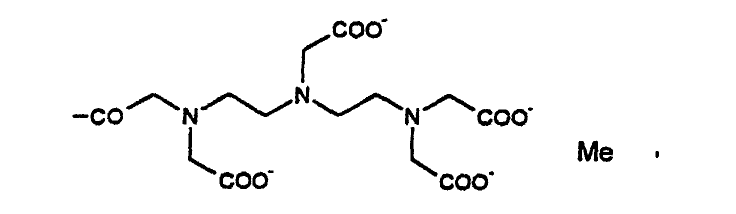 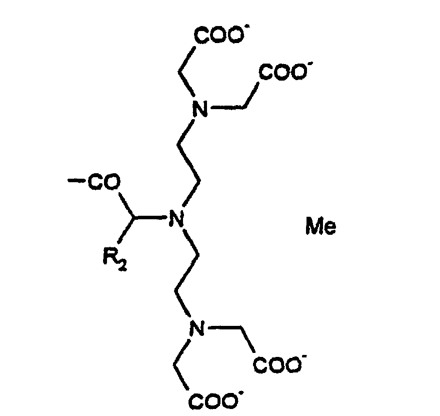 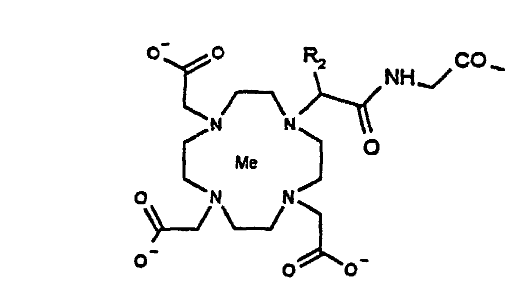 und 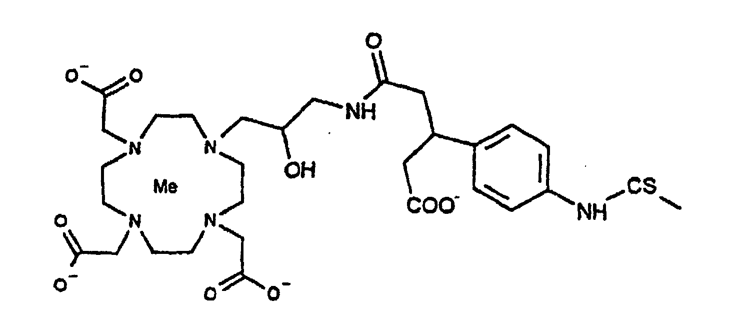
mit R₂ in der Bedeutung Wasserstoff, -(CH₂)₁₋₃-COOH, geradkettiges oder verzweigtes (C₁-C₄)-Alkyl, geradkettiges oder verzweigtes (C₁-C₄)-Hydroxyalkyl, Phenyl oder Benzyl, Me als Metallkation eines Elements der Ordnungszahl 21 bis 29, 39, 42, 44 oder 57-83 sowie gegebenenfalls Kationen anorganischer und /oder organischer Basen, Aminosäuren oder Aminosäureamide oder R₁ in der Bedeutung von 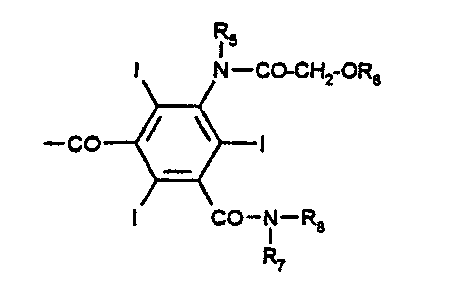 mit
   R₅ in der Bedeutung Wasserstoff oder C₁-C₂-Alkyl oder einer -(CH₂)₁₋₃-COOH- Gruppe,
   R₆ in der Bedeutung Wasserstoff oder Methyl und
   R₇, R₈ gleich oder verschieden in der Bedeutung Wasserstoff oder geradkettiges Alkyl mit 2-6 C-Atomen oder verzweigtkettiges Alkyl mit 3-6 C-Atomen, wobei beide Alkylreste mit 1-5 OH Gruppen substituiert sein können,
   R₃ für die Hydroxy- oder C₁-C₂-Alkoxy-Gruppe,
   R₄ für die Hydroxy-, Methyl - oder Methoxygruppe,
Y die Reste ―(CH₂)ₚ[O-CH₂-CH₂]ₒ, 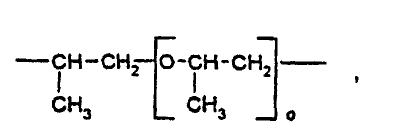 worin
   o die Zahlen 10-200, und
   p die Zahlen 2-20 bedeuten
   oder
Y den Rest -[CH₂]₁₀-CO-NH-[CH₂]₂- bedeutet, wobei im letzteren Fall R₄ für -N(R₁₀)-Z[N(R₁₀₎₂]₂ und R₃ für den Rest -NH R₉ steht,
   worin R₉ Wasserstoff, Benzyloxycarbonyl, 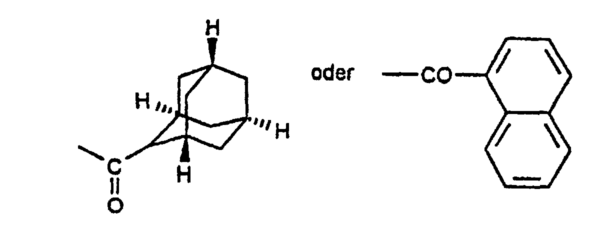
R₁₀ den Rest 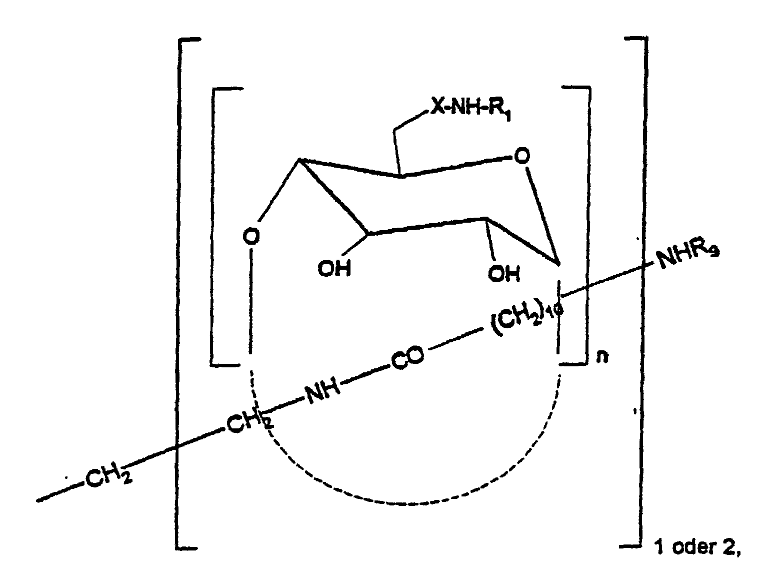 mit den oben angegebenen Bedeutungen für n, R₁ und R₉, und
Z den Rest 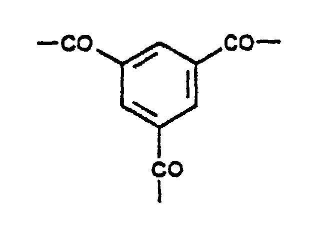 darstellen.

Für m ist der bevorzugte Bereich 5-30.
R₂ als geradkettiges (C₁-C₄)-Hydroxyalkyl bedeutet Hydroxymethyl, 2-Hydroxyethyl, 2- Hydroxy-propyl, 3-Hydroxy-propyl, 2-,3- oder 4-Hydroxy-n-butyl.
R₂ als verzweigtkettiges (C₃-C₄)-Alkyl bedeutet Isopropyl, sek.-Butyl, tert.-Butyl, Isobutyl. Unter R₂ als verzweigtkettigem (C₃-C₄)-Hydroxyalkyl versteht man folgende Reste:
1-Hydroxy-1-methylethyl, 2-Hydroxy-1-methylethyl, 1-Hydroxy-sek.-butyl,
1-Hydroxymethyl-n-propyl, 1-Hydroxy-isobutyl, 2-Hydroxyisobutyl, 3-Hydroxy-2-methyln-propyl und 2-Hydroxy-1.1-dimethyl-ethyl.

R₇ und R₈ als geradkettige oder verzweigte Alkylgruppen mit bis zu 6 C-Atomen, die durch 1-5 Hydroxygruppen substituiert sein können, bedeuten bevorzugt die bereits für R₂ genannten Gruppen sowie n-Pentyl, Isopentyl, Neopentyl, n-hexyl, sek.-Hexyl, Isohexyl, 2-Etyl-n-butyl. Als hydroxylierte Gruppen kommen hauptsächlich infrage:

-CH₂-CH₂-OH,

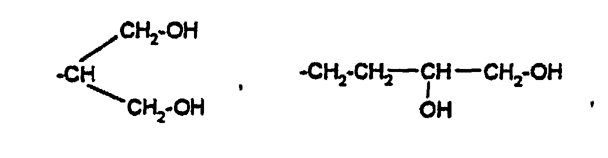

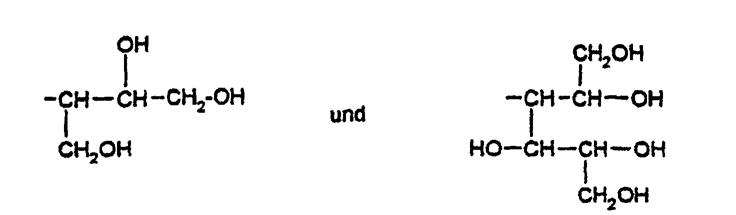

Für o kommt neben dem bereits genannten Zahlenbereich 10-200 bevorzugt der Zahlenbereich 25-100 in Betracht.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und
58 - 70. Geeignete Ionen sind beispielsweise das Chrom(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-, Mangan(II)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Räntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83 enthalten, geeignet sind; dies sind beispielsweise das Lanthan(III)-ion und die oben genannten Ionen der Lanthanidenreihe.
Die erfindungsgemäßen Pseudopolyrotaxan-Komplexe enthalten mindestens 12 Ionen eines Elements der oben genannten Ordnungszahl.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäure-amiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin,
N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die erfindungsgemäßen Verbindungen, die ein Molekulargewicht von 10.000 - 100.000, vorzugsweise 20.000 - 60.000 Da, besitzen, weisen die eingangs geschilderten gewünschten Eigenschaften auf. Sie enthalten die für ihre Verwendung benötigte große Anzahl von Metallionen im Komplex stabil gebunden.

Die neuen Pseudopolyrotaxane reichern sich in Gebieten mit erhöhter Gefäßpermeabilität, wie z.B. in Tumoren, an, erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA [Magnevist®], zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vasodilatativer Therapie, frühe Erkennung von minderperfundierten Gebieten (z.B. im Myokard), Angiographie bei Gefäßerkrankungen, und Erkennung und Diagnose von (sterilen oder infektiösen) Entzündungen. Außerdem sind die erfindungsgemäßen Pseudopolyrotaxan-Komplexe hervorragend zur Darstellung der Lymphgefäße (interstitielle und intravenöse Lymphographie) geeignet .

Als weitere Vorteile gegenüber extrazellulären Kontrastmitteln, wie z.B. Gd-DTPA [Magnevist®], muß die höhere Effektivität als Kontrastmittel für die Kernspintomographie (höhere Relaxivität) hervorgehoben werden, was zu einer deutlichen Reduktion der diagnostisch notwendigen Dosis führt. Gleichzeitig können die erfindungsgemäßen Kontrastmittel als Lösungen isoosmolar zum Blut formuliert werden und verringern dadurch die osmotische Belastung des Körpers, was sich in einer verringerten Toxizität der Substanz (höhere toxische Schwelle) niederschlägt. Geringere Dosen und höhere toxische Schwelle führen zu einer signifikanten Erhöhung der Sicherheit von Kontrastmittelanwendungen bei modernen bildgebenden Verfahren.

Im Vergleich zu anderen makromolekularen Kontrastmitteln auf der Basis von Kohlenhydraten, z.B. Dextran (Europäische Patentanmeldung, Publikations-Nr. 0 326 226), die - wie erwähnt - in der Regel nur ca. 5 % des signalverstärkenden paramagnetischen Kations tragen, weisen die erfindungsgemäßen Pseudopolyrotaxan-Komplexe einen Gehalt von in der Regel ca. 10-20% des paramagnetischen Kations auf. Somit bewirken die erfindungsgemäßen Makromoleküle pro Molekül eine sehr viel höhere Signalverstärkung, was gleichzeitig dazu führt, daß die zur Kernspintomographie notwendige Dosis erheblich kleiner ist.

Mit den erfindungsgemäßen Pseudopolyrotaxan-Komplexen ist es gelungen, hochmolekulare Kontrastmittel zur Verfügung zu stellen, die überraschenderweise vollständig eliminiert werden, obwohl das durchschnittliche Molkulargewicht z.T. deutlich über der Nierenfiltrationsschwelle liegt.

Im Vergleich zu den anderen erwähnten Polymer-Verbindungen des Stands der Technik zeichnen sich die erfindungsgemäßen Komplexe durch verbessertes Ausscheidungsverhalten, höhere Wirksamkeit, größere Stabilität und/oder bessere Verträglichkeit aus.

Ein weiterer Vorteil der vorliegenden Erfindung liegt darin, daß nunmehr Komplexe mit hydrophilen oder lipophilen, makrocyclischen oder offenkettigen, niedermolekularen oder hochmolekularen Liganden zugänglich geworden sind. Dadurch ist die Möglichkeit gegeben, Verträglichkeit und Pharmakokinetik dieser Polymer-Komplexe durch chemische Substitution zu steuern.

Die Herstellung der erfindungsgemäßen Pseudopolyrotaxan-Komplexe erfolgt dadurch, daß man Verbindungen der allgemeinen Formel II, 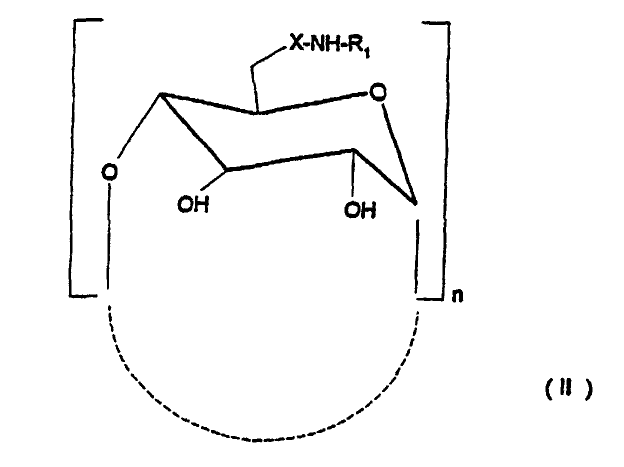 worin X, R₁ und n die oben angegebenen Bedeutungen haben, mit Verbindungen der Formel III

R₃-Y-R₁₁ (III),

worin Y und R₃ die oben angegebenen Bedeutungen haben und R₁₁ Hydroxy, Methyl, Methoxy oder, falls Y für -(CH₂)₁₀-CO-NH (CH₂)₂ - steht, den Rest -N(R₁₂)Z [N(R₁₂)₂]₂ bedeutet,
R₁₂ den Rest -(CH₂)₂-NH-CO-(CH₂)₁₀-NHR₉ mit R₉ in der oben angebenen Bedeutung darstellt, umsetzt wie z.B. in J. Am. Chem. Soc. 116, 3192 (1994) beschrieben.

Die Ausgangssubstanzen der allgemeinen Formel III sind entweder käuflich (z.B. Aldrich) zu erhalten oder analog dem in Beispiel 3 offenbarten Verfahren herzustellen. Die Edukte der allgemeinen Formel II werden erhalten analog den in den Beispielen offenbarten Verfahren durch Verwendung von literaturbekannten Cyclodextrin-oligo-aminen oder durch Verwendung von Aminosäureestem der Cyclodextrine, die amidisch mit Carbonsäure-aktivierten Komplexen oder Komplexliganden oder trijodierten Benzolderivaten, wie für R₁ angegeben, umgesetzt werden (s.z.B. DE 39 38992, WO 93/10824, DE 4 4258 57).

Die Reinigung der erhaltenen Pseudopolyrotaxan-Komplexe erfolgt gegebenenfalls nach Einstellung des pH-Wertes durch Zusatz einer Säure oder Base auf pH 6 bis 8, bevorzugt ca. 7, vorzugsweise durch Ultrafiltration mit Membranen geeigneter Porengröße (z.B. Amicon® XM30, Amicon® YM10, Amicon® YM3) oder Gelfiltration an z.B. geigneten Sephadex®-Gelen.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die korrespondierende Calcium-Pseudorotaxan-Komplexe) oder - falls erforderlich - Elektrolyte wie zum Beispiel Natriumchlorid oder - falls erforderlich - Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj®] und/oder Aromastoff(en) zur Geschmackskorrektur [zum Beispiel ätherischen Ölen] gemischt.

Die erfindungsgemäßen pharmazeutischen Mittel enthalten vorzugsweise 1µMol - 1 Mol/l des Komplexsalzes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung für die NMR- und Röntgen-Diagnostik in Form ihrer Komplexe mit den Ionen der Elemente mit den Ordnungszahlen 21-29, 39, 42, 44 und 57-83.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001 - 5 mMol/kg, vorzugsweise 0,005 - 0,5 mMol/kg, dosiert. Details der Anwendung werden zum Beispiel in H.-J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Im allgemeinen werden die erfindungsgemäßen Mittel für die Anwendung als Röntgenkontrastmittel in Analogie zu z.B. Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thum, E. Bücheler "Einführung in die Röntgendiagnostik", G. Thieme, Stuttgart, New York (1977) diskutiert.

Insgesamt ist es gelungen, neue Metallkomplex- und jodhaltige Pseudopolyrotaxane zu synthetisieren, die neue Möglichkeiten in der Diagnostik erschließen.

Die nachfolgenden Beispiele dienen zur näheren Erläuterung des Erfindungsgegenstands:

### Beispiel 1

### a) Hexa-(Gd-DTPA-monoamid)-Derivat des 6,6',6",6"',6"",6""'-Hexaamino-6,6',6",6"',6"",6""'hexadeoxy-α-cyclodextrins

1,26 g (1 mmol)6,6',6",6"',6"",6""'-Hexaamino-6,6',6",6"',6"",6""-hexadeoxy-α-cyclodextrinhexahydrochlorid [J. Boger, R.J. Corcoran und J.-M. Lehn, Helv. Chim. Acta 61, 2190-2218 (1978)] werden in 80 ml Wasser gelöst. Innerhalb einer halben Stunde werden anschließend 7,26 g (18 mmol) N³-(2,6-Dioxomorpholinoethyl)-N⁶-(ethoxycarbonylmethyl)-3,6-diazaoctandisäure (Beispiel 13a der EP 0331 616) portionsweise in fester Form zugegeben, wobei der pH-Wert durch Zugabe von 1N Natronlauge bei 7,5-8 gehalten wird. Dann gibt man zur Verseifung der Ethylester weitere 1N Natronlauge bis zu einem pH > 13 zu und läßt 3 Stunden bei diesem pH rühren. Anschließend wird die alkalische Lösung mit Amberlite IR 120 (H⁺-Form) auf pH 5 gestellt, vom Ionenaustauscher abgesaugt und mit 4,75 g GdCl₃ (18 mmol) versetzt, 30 Minuten bei 80°C gerührt, mit 1N Natronlauge auf pH 7,2 eingestellt und die entstandene Lösung ultrafiltriert (AMICON® YM 1-Membran). Die von niedermolekularen Bestandteilen befreite Lösung wird schließlich gefriergetrocknet. Man erhält 4,38 g (94,4 % d. Th.) farbloses, flockiges Pulver.
H₂O-Gehalt (Karl-Fischer): 7,8 %
Gd-Bestimmung (AAS): 20,1 %

| Elementaranalyse (bezogen auf wasserfreie Substanz): | | | | | |
|---|---|---|---|---|---|
| ber. | C 33,71 | H 3,96 | N 7,86 | Gd 22,06 | Na 3,23 |
| gef. | C 33,97 | H 4,22 | N 7,69 | Gd 21,48 | Na 2,80 |

### b) Polyrotaxan aus Methoxypolyethylenglykol und dem im Beispiel 1a beschriebenen α-Cyclodextrin-hexa-Gd-Komplex

928 mg (0,2 mmol) des in Beispiel 1a beschriebenen Hexa-(GdDTPA-monoamid)-Derivats des 6,6',6",6"',6"",6""'-hexaamino-6,6',6",6"',6"",6""'-hexadeoxy-α-cyclodextrins werden in 5 ml Wasser gelöst und unter Rühren mit 10 mg (5 µmol) Methoxypolyethylenglykol (Sigma, M=2000) versetzt. Die Lösung wird 10 Minuten im Ultraschallbad beschallt und anschließend über Nacht bei Raumtemperatur gerührt. Zur Abtrennung von niedermolekularen Bestandteilen wird über eine 15 ml AMICON® Zentrifugationseinheit centriplus-3® (cut-off 3.000 Dalton) filtriert und anschließend gefriergetrocknet. Man erhält 430 mg farbloses flockiges Lyophilisat.
H₂O-Gehalt (Karl-Fischer): 4,7 %
Gd-Bestimmung (AAS): 20,3 %

| Elementaranalyse (bezogen auf wasserfreie Substanz): | | | | | |
|---|---|---|---|---|---|
| ber. | C 34,13 | H4,06 | N 7,70 | Gd 21,62 | Na 3,16 |
| gef. | C 34,07 | H 4,34 | N 7,46 | Gd 21,33 | Na 2,87 |

Photonenkorrelationsspektroskopie zeigt im numerischen Mittel der hydrodynamischen Teilchengröße Werte von > 30 nm.

### Beispiel 2

### a) 6,6',6",6"',6"",6""',6"""-Heptaamino-6,6',6"',6"",6""',6"""-heptadeoxy-β-cyclodextrinheptahydrochlorid

1,90 g (1 mmol) 6,6',6",6"',6"",6""',6"""-Heptaamino-6,6',6",6"',6"",6""',6"""-heptadeoxy-β-cyclodextrin-2,2',2",2'",2"",2""',2""",3,3',3",3'",3"",3"'",3"""-tetradecaacetat [J. Boger et al., Helv. Chim. Acta 61, 2190-2218 (1978) werden in Dioxan/Methanol (10:1) gelöst und nach Zugabe von 14 ml (28 mmol) 2 N Natronlauge 2 Stunden bei Raumtemperatur gerührt und anschließend mit verdünnter Salzsäure auf pH 7 gestellt. Die neutralisierte Lösung wird im Vakuum zum Trockne eingedampft, der Rückstand nacheinander mit Chloroform und Wasser gewaschen und wieder zur Trockne eingedampft. Das erhaltene heptakis (6-azido-6-deoxy)-β-CD wird unter Stickstoff in Dioxan/Methanol (5:1) suspendiert, mit 5,25 g (20 mmol) Triphenylphosphin versetzt, die entstandene Lösung eine Stunde bei Raumtemperatur gerührt und anschließend mit konz. Ammoniak versetzt. Nach Rühren über Nacht wird im Vakuum zur Trockne eingedampft, mit Wasser aufgenommen und mit 1 N Salzsäure auf pH 6 gestellt und die Lösung gefriergetrocknet. Man erhält 1,33 g farbloses flockiges Pulver (85 % d. Th.).
H₂O-Gehalt (Karl-Fischer): 11,5 %

| Elementaranalyse (bezogen auf wasserfreie Substanz): | | | | |
|---|---|---|---|---|
| ber. | C 36,47 | H 6,12 | N 7,09 | Cl 17,94 |
| gef. | C 36,77 | H 6,21 | N 6,86 | Cl 17,71 |

### b) Hepta-(Gd-DTPA-monoamid)-Derivat des 6,6',6",6"',6"",6""',6"""-Heptaamino-6,6',6",6"',6"",6""',6"""-heptadeoxy-β-cyclodextrins

782 mg (0,5 mmol) des im vorstehenden Beispiel 2a beschriebenen heptakis (6-amino-6-deoxy)-β-CD-heptahydrochlorids werden in 80 ml Wasser gelöst. Innerhalb einer halben Stunde werden anschließend 4,24 g (10,5 mmol) N³-(2,6-Dioxomorpholinoethyl)-N⁶-(ethoxycarbonylmethyl)-3,6-diazaoctandisäure (Beispiel 13a der EP 0331 616) portionsweise in fester Form zugegeben, wobei der pH-Wert durch Zugabe von 1N Natronlauge bei 7,5-8 gehalten wird. Dann gibt man zur Verseifung der Ethylester weitere 1N Natronlauge bis zu einem pH > 13 zu und läßt 3 Stunden bei diesem pH rühren. Anschließend wird die alkalische Lösung mit Amberlite IR 120 (H⁺-Form) auf pH 5 gestellt, vom Ionenaustauscher abgesaugt und mit 2,77 g GdCl₃ (10,5 mmol) versetzt, 30 Minuten bei 80°C gerührt, mit 1 N Natronlauge auf pH 7,2 eingestellt und die entstandene Lösung ultrafiltriert (AMICON® YM 1-Membran). Die von niedermolekularen Bestandteilen befreite Lösung wird schließlich gefriergetrocknet. Man erhält 2,49 g (91,1 % d. Th.) farbloses, flockiges Pulver.
H₂O-Gehalt (Karl-Fischer): 8,6 %
Gd-Bestimmung (AAS): 19,9 %

| Elementaranalyse (bezogen auf wasserfreie Substanz): | | | | | |
|---|---|---|---|---|---|
| ber. | C 33,71 | H 3,96 | N 7,86 | Gd 22,06 | Na 3,23 |
| gef. | C 33,85 | H 4,11 | N 7,70 | Gd 21,83 | Na 3,06 |

### c) Polyrotaxan aus Poly-(1,2-propandiol) und dem in Beispiel 2b beschriebenen β-Cyclodextrin-hepta-Gd-Komplex

1,09 g (0,2 mmol) des in Beispiel 2b beschriebenen Hepta-(GdDTPA-monoamid)-Derivats des 6,6',6",6"',6"",6""',6"""-heptaamino-6,6',6",6"',6"",6""',6"""-heptadeoxy-β-cyclodextrins werden in 10 ml Wasser gelöst und unter Rühren mit 20 µl (0,02 mmol) Poly-(1,2-propandiol) (Aldrich, M_{w}=1000) versetzt, wobei sich die Polymertröpfchen allmählich auflösen. Die Lösung wird 10 Minuten im Ultraschallbad beschallt und anschließend über Nacht bei Raumtemperatur gerührt. Zur Abtrennung von niedermolekularen Bestandteilen wird über eine 15 ml AMICON® Zentrifugationseinheit centriplus-3® (cut-off 3.000 Dalton) filtriert und anschließend gefriergetrocknet. Man erhält 620 mg farbloses flockiges Lyophilisat.
H₂O-Gehalt (Karl-Fischer): 7,9 %
Gd-Bestimmung (AAS): 19,7 %

| Elementaranalyse (bezogen auf wasserfreie Substanz): | | | | | |
|---|---|---|---|---|---|
| ber. | C 34,35 | H 4,11 | N 7,68 | Gd 21,56 | Na 3,15 |
| gef. | C 34,09 | H 3,86 | N 7,73 | Gd 21,70 | Na 2,94 |

Photonenkorrelationsspektroskopie zeigt im numerischen Mittel der hydrodynamischen Teilchengröße Werte von > 30 nm.

### Beispiel 3

### a) Bis[2-(benzyloxycarbonylamino)-ethyl]-amin

5,15 g (50 mmol) Diethylentriamin und 13,9 ml (100 mmol) Triethylamin werden in Dichlormethan gelöst und bei -20°C mit 16,1 g Benzylcyanformiat (Fluka) in Dichlormethan versetzt und anschließend über Nacht bei Raumtemperatur gerührt. Nach Beendigung der Reaktion wird im Abzug eingedampft, der Rückstand in Diethylether aufgenommen, die organische Phase mit Natriumcarbonatlösung gewaschen und mit Natriumsulfat getrocknet. Das Filtrat wird mit Hexan versetzt, der Niederschlag abfiltriert und getrocknet.
Ausbeute: 16,34 g (88 % d. Th.)

| Elementaranalyse: | | | |
|---|---|---|---|
| ber. | C 64,67 | H 6,78 | N 11,31 |
| gef. | C 64,48 | H 6,85 | N 11,19 |

### b) N,N,N',N',N",N"-Hexakis[2-(benzyloxycarbonylamino)-ethyl]-trimesinsäuretriamid

1,33 g (5 mmol) Trimesinsäure-trichlorid (Aldrich) und 3,47 ml (25 mmol) Triethylamin werden in Dimethylformamid (DMF) gelöst und bei 0°C mit 6,5 g (17,5 mmol) des in Beispiel 3a beschriebenen Amins versetzt und anschließend über Nacht bei Raumtemperatur gerührt. Die Lösung wird im Vakuum eingedampft und der Rückstand mit Ethylacetat an Kieselgel chromatographiert.
Ausbeute: 3,94 g (62 % d. Th.)

| Elementaranalyse: | | | |
|---|---|---|---|
| ber. | C 65,24 | H 5,95 | N 9,92 |
| gef. | C 65,47 | H 6,03 | N 9,82 |

### c) N,N,N',N',N",N"-Hexakis[14-(benzyloxycarbonylamino)-4-oxo-3-aza-tetradecyl] trimesinsäuretriamid

1,27 g (1 mmol) des im Beispiel 3b beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexaamin-hydrobromid mit Ether gewaschen, im Vakuum getrocknet und ohne weitere Reinigung in die weiter unten beschriebene Reaktion eingesetzt.
Ausbeute: 0,95 g (quantitativ)

2,52 g (7,5 mmol) N-Benzyloxycarbonylamino-undecansäure (H.N. Rydon et al., J. Chem. Soc. 1965, 4246-53), 1,2 g (7,5 mmol) 1-Hydroxybenzotriazol und 2,4 g (7,5 mmol) 2-(1H-Benzotriazol-1-yl)-1,1,3,3-tetramethyluronium tetrafluorborat (TBTÜ; Peboc Limited, UK) werden in DMF gelöst und 15 Minuten gerührt. Diese Lösung wird anschließend mit 5,16 ml (30 mmol) N-Ethyldiisopropylamin und mit 0,95 g (1 mmol) des oben beschriebenen Hexa-aminhydrobromids versetzt und über Nacht bei Raumtemperatur gerührt. Nach Beendigung der Reaktion wird im Vakuum eingedampft und der Rückstand mit Ethylacetat/Ethanol (2:1) an Kieselgel chromatographiert.
Ausbeute: 1,92 g (81 % d. Th.)

| Elementaranalyse: | | | |
|---|---|---|---|
| ber. | C 68,41 | H 8,55 | N 8,86 |
| gef. | C 68,20 | H 8,72 | N 8,74 |

### d) Polyrotaxan aus N,N,N',N',N",N"-Hexakis[14-(benzyloxycarbonylamino)-4-oxo-3-azatetradecyl] trimesinsäuretriamid und dem in Beispiel 2b beschriebenen β-Cyclodextrin-hepta-Gd-Komplex

1,09 g (0,2 mmol) des in Beispiel 2b beschriebenen Hepta-(GdDTPA-monoamid)-Derivats des 6,6',6",6"',6"",6""',6"""-heptaamino-6,6',6",6"',6"",6""',6"""-heptadeoxy-β-cyclodextrins werden in 10 ml Wasser gelöst und unter Rühren mit 47 mg (0,02 mmol) des im vorstehenden Beispiel 3c beschriebenen Hexameren versetzt. Die Suspension wird 10 Minuten im Ultraschallbad beschallt und anschließend über Nacht bei Raumtemperatur gerührt. Zur Abtrennung von niedermolekularen Bestandteilen wird über eine 15 ml AMICON® Zentrifugationseinheit centriplus-10® (cut-off 10.000 Dalton) filtriert und anschließend gefriergetrocknet. Man erhält 1,13 g farbloses flockiges Lyophilisat.
H₂O-Gehalt (Karl-Fischer): 8,6 %
Gd-Bestimmung (AAS): 19,7 %

| Elementaranalyse (bezogen auf wasserfreie Substanz, berechnet als 14-Rotaxan, d.h. durchschnittlich mit 2,33 β-CD's pro Arm besetzt): | | | | | |
|---|---|---|---|---|---|
| ber. | C 34,84 | H 4,11 | N 7,89 | Gd 21,34 | Na 3,12 |
| gef. | C 34,76 | H 3,89 | N 7,62 | Gd 21,05 | Na 2,90 |

### Beispiel 4

### a) 10-[5-(2-Carboxyphenyl)-2-Hydroxy-5-oxo-4-aza-pentyl]-1,4,7-tris(carboxymethyl)-1,4,7,10-tetraazacyclododecan

50 g (144,3 mmol) 1,4,7-Tris(carboxymethyl)-1,4,7,10-tetraazacyclododecan (DO3A) werden in 250 ml Wasser gelöst und der pH-Wert mit 5 N Natronlauge auf pH 13 eingestellt. Dann wird innerhalb einer Stunde eine Lösung aus 38,12 g (187,6 mmol) N(2,3-Epoxypropyl)-phthalimid (Aldrich) in 100 ml Dioxan zugetropft, 24 Stunden bei 50°C gerührt und der pH-Wert durch Zugabe von 5 N Natronlauge bei pH 13 gehalten. Die Lösung wird mit 10%iger Salzsäure auf pH 2 gestellt und im Vakuum zur Trockne eingedampft. Der Rückstand wird in etwas Wasser gelöst und an einer Ionenaustauschersäule (Reillex®= Poly-(4-vinyl)-pyridin (man eluiert mit Wasser) gereinigt. Die Hauptfraktionen werden im Vakuum eingedampft und der Rückstand durch Chromatographie an RP-18 (LiChroPrep®/Laufmittel: Gradient aus Tetrahydrofuran/Methanol/Wasser) endgereinigt. Nach Eindampfen der Hauptfraktionen erhält man 63,57 g (71 % d. Th.) eines amorphen Feststoffes.
Wassergehalt: 8,5 %

| Elementaranalyse (bezogen auf wasserfreie Substanz): | | | |
|---|---|---|---|
| ber. | C 52,90 | H 6,57 | N 12,34 |
| gef. | C 52,65 | H 6,68 | N 12,15 |

### b) 10-(3-Amino-2-hydroxypropyl)-1,4,7-tris(carboxymethyl)-1,4,7,10-tetraazacyclododecan

50 g (88,1 mmol) der Titelverbindung aus Beispiel 4a werden in 300 ml konz. Salzsäure 24 Stunden unter Rückfluß erhitzt. Man dampft zur Trockne ein, löst den Rückstand in etwas Wasser und reinigt an einer Ionenaustauschersäule (Reillex®=Poly-(4-vinyl)pyridin (man eluiert mit Wasser). Die Hauptfraktionen werden zur Trockne eingedampft.
Ausbeute: 39,0 g (95 % d. Th.) eines glasigen Feststoffes
Wassergehalt: 10,3 %

| Elementaranalyse (bezogen auf wasserfreie Substanz): | | | |
|---|---|---|---|
| ber. | C 48,68 | H 7,93 | N 16,70 |
| gef. | C 48,47 | H 8,09 | N 16,55 |

### c) Gadolinium-Komplex des 10-(3-Amino-2-hydroxy-propyl)-1,4,7-tris(carboxymethyl)-1,4,7,10-tetraazacyclododecans

38 g (90,6 mmol) der Titelverbindung aus Beispiel 4b werden in 300 ml Wasser gelöst und 16,42 g (45,3 mmol) Gadoliniumoxid zugesetzt. Man erwärmt 3 Stunden auf 90°C. Die abgekühlte Lösung wird mit je 5 ml saurem Ionenaustauscher (IR-120/H⁺-Form) und 5 ml basischem Austauscher (IRA-410/OH⁻-Form) eine Stunde bei Raumtemperatur gerührt. Man filtriert vom Austauscher ab. Gefriertrocknung des Filtrats liefert 57,23 g (98 % d.Th.) eines amorphen Feststoffes.
Wassergehalt: 11,3 %

| Elementaranalyse (bezogen auf wasserfreie Substanz): | | | | |
|---|---|---|---|---|
| ber. | C 35,59 | H 5,27 | Gd 27,41 | N 12,21 |
| gef. | C 35,32 | H 5,38 | Gd 27,20 | N 12,31 |

### d) Gadolinium-Komplex des 10-[7-(4-Nitrophenyl)-2-hydroxy-5-oxo-7-(carboxymethyl)-4-azaheptyl]-1,4,7-tris(carboxymethyl)-1,4,7,10-tetraazacyclododecans

Zu 20 g (34,86 mmol) der Titelverbindung aus Beispiel 4c in 200 ml Dimethylformamid/ 20 ml Triethylamin gibt man 9,84 g (41,8 mmol) 3-(4-Nitrophenyl)-glutarsäureanhydrid (Journal of Org. Chem., Vol. 26, 3856 (1961)) und rührt über Nacht.bei Raumtemperatur. Man dampft im Vakuum zur Trockne ein. Der Rückstand wird aus Isopropanol/Essigsäure 95:5 umkristallisiert. Ausbeute: 27,46 g (94 % d. Th.) eines gelblichen Feststoffes
Wassergehalt: 3,4 %

| Elementaranalyse (bezogen auf wasserfreie Substanz): | | | | |
|---|---|---|---|---|
| ber. | C 41,58 | H 4,86 | Gd 19,44 | N 10,39 |
| gef. | C 41,38 | H 4,97 | Gd 19,28 | N 10,17 |

### e) Gadolinium-Komplex des 10-[7-(4-Aminophenyl)-2-hydroxy-5-oxo-7-(carboxymethyl)-4-azaheptyl]-1,4,7-tris(carboxymethyl)-1,4,7,10-tetraazacyclododecans

25 g (30,9 mmol) der Titelverbindung aus Beispiel 4d werden in 250 ml Methanol gelöst und 5 g Palladium-Katalysator (10 % Pd auf C) zugegeben. Man hydriert über Nacht bei Raumtemperatur. Der Katalysator wird abfiltriert und das Filtrat im Vakuum zur Trockne eingedampft.
Ausbeute: 24,07 g (97 % d. Th.) eines cremefarbenen Feststoffes
Wassergehalt: 3,0 %

| Elementaranalyse (bezogen auf wasserfreie Substanz): | | | | |
|---|---|---|---|---|
| ber. | C 43,18 | H 5,31 | Gd 20,19 | N 10,79 |
| gef. | C 43,27 | H 5,48 | Gd 20,02 | N 10,61 |

### f) Gadolinium-Komplex des 10-[7-(4-Isothiocyanatophenyl)-2-hydroxy-5-oxo-7-(carboxymethyl)-4-aza-heptyl]-1,4,7-tris(carboxymethyl)-1,4,7,10-tetraazacyclododecans

15 g (19,26 mmol) der Titelverbindung aus Beispiel 4e werden in 100 ml Wasser gelöst und 6,64 g (57,8 mmol) Thiophosgen in 50 ml Chloroform zugegeben. Man rührt 1 Stunde bei 50°C. Man kühlt auf Raumtemperatur, trennt die organische Phase ab und schüttelt die wässrige Phase 2 mal mit 100 ml Chloroform aus. Die wässrige Phase wird zur Trockne eingedampft und der Rückstand in 100 ml Isopropanol bei Raumtemperatur ausgerührt. Der Feststoff wird abfiltriert und mit Ether gewaschen. Nach Trocknen über Nacht im Vakuum (40°C) erhält man 15,9 g (98 % d. Th.) eines cremefarbenen Feststoffes.
Wassergehalt: 3,5 %

| Elementaranalyse (bezogen auf wasserfreie Substanz): | | | | | |
|---|---|---|---|---|---|
| ber. | C 42,43 | H 4,79 | Gd 19,15 | N 10,24 | S 3,91 |
| gef. | C 42,23 | H 4,90 | Gd 19,01 | N 10,05 | S 3,96 |

### g) 6,6',6",6"',6"",6""'-Hexakis(N-Fluorenylmethoxycarbonyl-alanyl)-α-cyclodextrin

2,08 g (2 mmol; Wassergehalt 7 %) α-Cyclodextrin werden in Dimethylformamid gelöst, mit Benzol versetzt und azeotrop entwässert. Es wird anschließend auf 0°C gekühlt, 1,32 ml (12 mmol) N-Methylmorpholin zugegeben und nach Zugabe von 4,05 g (12 mmol) N-Fluorenylmethoxycarbonyl-alanin-N-carbonsäureanhydrid, Fmoc-Ala-NCA (Propeptide, SNPE GmbH, Frankfurt/M.) wird über Nacht bei Raumtemperatur gerührt. Die Lösung wird dann im Vakuum eingedampft, der Rückstand mit Wasser gewaschen und schließlich aus Essigester umkristallisiert.
Ausbeute: 5,08 g (93 % d. Theorie)

| Elementaranalyse (bezogen auf wasserfreie Substanz): | | | |
|---|---|---|---|
| ber. | C 63,29 | H 5,53 | N 3,08 |
| gef. | C 63,14 | H 5,70 | N 2,98 |

### h) Hexa-Thioharnstoff-Konjugat aus Hexakis-(O-alanyl)-α-CD mit dem Gadolinium-Komplex des 10-[7-(4-Isothiocyanatophenyl)-2-hydroxy-5-oxo-7-(carboxymethyl)-4-azaheptyl]-1,4,7-tris(carboxymethyl)-1,4,7,10-tetraazacyclododecans

2,78 g (1 mmol) des im vorstehenden Beispiel 4g beschriebenen Hexakis-[O-(Fmoc-alanyl)]-α-CD's werden in Dimethylformamid gelöst und mit 4 g 4-Dimethylaminopyridin versetzt. Nach 15 Minuten Rühren bei Raumtemperatur werden 5,31 g (6,3 mmol) des in Beispiel 4f beschriebenen Isothiocyanats zugegeben und über Nacht gerührt. Die Lösung wird anschließend im Vakuum eingeengt, mit Wasser aufgenommen, auf pH 7 eingestellt und über eine AMICON® Ultrafiltrationsmembran YM 1 von niedermolekularen Anteilen befreit. Nach beendeter Ultrafiltration wird mit verdünnter Natronlauge nochmals auf pH 7 eingestellt, das Retentat eingefroren und gefriergetrocknet. Man erhält 6,78 g (97 % d. Theorie) leicht gelbliches flockiges Pulver. Eine analytische Probe-Anfärbung mit Ninhydrin zeigt, daß keine freien Aminogruppen mehr in dem Thio-Harnstoff-Konjugat vorhanden sind.
Wassergehalt (Karl-Fischer): 8,3 %
Gd-Bestimmung (AAS): 13,2 %

| Elementaranalyse (bezogen auf wasserfreie Substanz): | | | | | | |
|---|---|---|---|---|---|---|
| ber. | C 42,41 | H 4,96 | Gd 14,61 | N 9,11 | Na 2,14 | S 2,98 |
| gef. | C 42,25 | H 5,10 | Gd 14,26 | N 9,01 | Na 1,79 | S 2,66 |

### i) Polyrotaxan aus Polyethylenglykol und dem in Beispiel 4h beschriebenen Hexa-Gd-Komplex des α-Cyclodextrins

1,40 g (0,2 mmol) des in Beispiel 4h beschriebenen Hexa-Gd-Komplexes des α-Cyclodextrins werden in 10 ml Wasser gelöst und unter Rühren mit 10 mg (6,9 µmol) Polyethylenglykol (Sigma; M_{w} = 1450) versetzt. Die Lösung wird 10 Minuten im Ultraschallbad beschallt und anschließend über Nacht bei Raumtemperatur gerührt. Zur Abtrennung von niedermolekularen Bestandteilen wird über eine 15 ml AMICON® Zentrifugationseinheit centriplus-3® filtriert und anschließend gefriergetrocknet. Man erhält 560 mg leicht gelbliches flockiges Lyophilisat.
H₂O-Gehalt (Karl-Fischer): 9,3 %
Gd-Bestimmung (AAS): 12,9 %

| Elementaranalyse (bezogen auf wasserfreie Substanz): | | | | | | |
|---|---|---|---|---|---|---|
| ber. | C 42,57 | H 5,02 | Gd 14,42 | N 8,99 | Na 2,11 | S 2,94 |
| gef. | C 42,33 | H 5,00 | Gd 14,20 | N 9,09 | Na 1,98 | S 2,77 |

Photonenkorrelationsspektroskopie zeigt im numerischen Mittel der hydrodynamischen Teilchengröße Werte > 30 nm.

### Beispiel 5

### a) Hexaamid-Derivat der 2,4,6-Trijod-3-N-(2-hydroxyethyl)-5-(hydroxy)acetatamidoisophthalsäure mit 6,6',6",6"',6"",6""'-Hexaamino-6,6',6",6"',6"",6"'"-hexadeoxy-α-cyclodextrin

Zu 1,26 g (1 mmol) 6,6',6",6"',6"",6""'-Hexaamino-6,6',6",6"',6"",6""'-hexadeoxy-α-cyclodextrinhexahydrochlorid [J. Boger, RJ. Corcorcan und J.-M. Lehn, Helv. Chim. Acta 61, 2190-2218 (1978)] in 40 ml N,N-Dimethylacetamid gibt man 2,02 g (20 mmol) Triethylamin und 5,03 g (6,60 mmol) des Säurechlorids der 2,4,6-Trijod-3-N-(2-acetoxyethyl)-5-acetoxy-acetamidoisophthalsäure [Guerbet S.A., WO 93/10824]. Man rührt 24 Stunden bei Raumtemperatur. Es wird im Vakuum zur Trockne eingedampft, der Rückstand in 200 ml Methylenchlorid gelöst und 2 mal mit je 100 ml 5 %iger aqu. Salzsäure und 100 ml 5 %iger Natriumcarbonatlösung gewaschen. Die organische Phase wird im Vakuum zur Trockne eingedampft, der Rückstand in 200 ml Methanol gelöst und bei 0°C Ammoniak (Gas) bis zur Sättigung eingeleitet. Man rührt 6 Stunden bei 0°C, anschließend 1 Stunde bei 40°C. Man dampft zur Trockne ein und chromatographiert den Rückstand an einer RP-18 Säule (Laufmittel: Gradient aus Wasser/Acetonitril/n-Propanol).
Ausbeute: 4,1 g (85 % d. Th.) eines farblosen Feststoffes
Wassergehalt: 1,2 %

| Elementaranalyse (bezogen auf wasserfreie Substanz): | | | | |
|---|---|---|---|---|
| ber. | C 26,92 | H 2,51 | N 5,23 | J 47,41 |
| gef. | C 26,71 | H 2,70 | N 5,05 | J 47,19 |

### b) Polyrotaxan aus Methoxypolyethylenglycol (M= 2000) und dem unter Beispiel 5a beschriebenen a-Cyclodextrin-hexa-amids

936,7 mg (0,2 mmol) der Titelverbindung aus Beispiel 5a werden in 5 ml Wasser gelöst und unter Rühren mit 10 mg (5 µmol) Methoxypolyethylenglycol (Sigma, M= 2000) versetzt. Die Lösung wird 10 Minuten im Ultraschallbad beschallt und anschließend über Nacht bei Raumtemperatur gerührt. Zur Abrennung von niedermolekularen Bestandteilen wird über eine 15 ml AMICON® Zentrifugationseinheit centriplus-3® (cut-off 3.000 Dalton) filtriert und anschließend gefriergetrocknet. Man erhält 389 mg farbloses flockiges Lyophilisat.
Wassergehalt: 3,9 %

| Elementaranalyse (bezogen auf wasserfreie Substanz): | | | | |
|---|---|---|---|---|
| ber. | C 29,23 | H 3,07 | N 4,79 | J 43,44 |
| gef. | C 29,01 | H 3,19 | N 4,61 | J 43,28 |

Photonenkorrelationsspektroskopie zeigt im numerischen Mittel der hydrodynamischen Teilchengröße Werte von > 25 nm.

## Patentansprüche

1. Pseudopolyrotaxane enthaltend als bildgebende Komponente für die MRT- und Röntgendiagnostik Metallkomplex oder jodhaltige Benzolderivate.

2. Pseudopolyrotaxane der Formel I 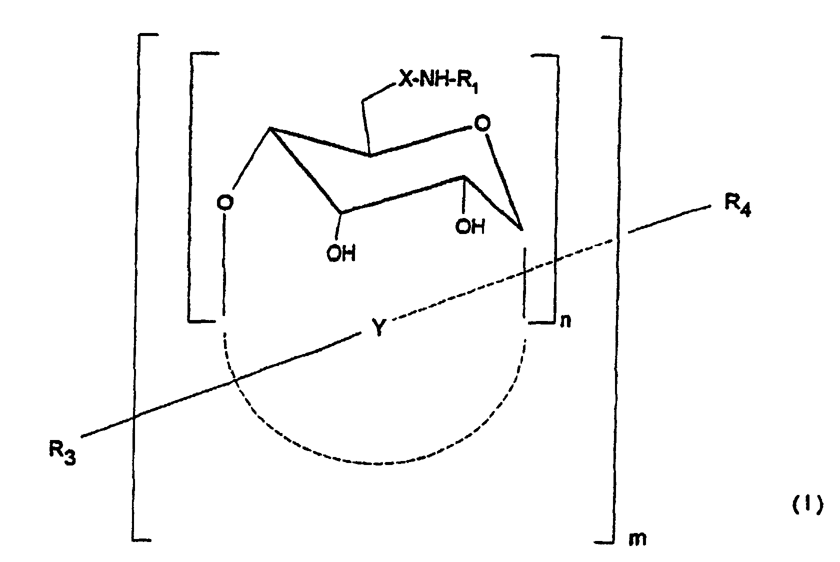 worin
n die Zahlen 6, 7 oder 8,
m die Zahlen 2 bis 50,
X eine direkte Bindung oder den Rest -O-CO-CH(CH₃)-
R₁ die kontrastgebenden Reste 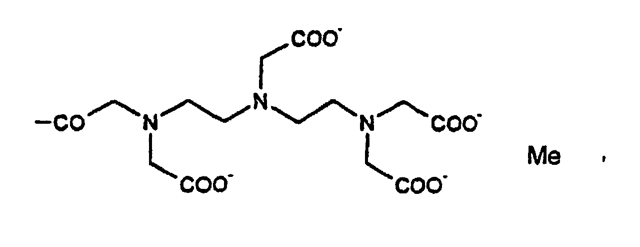 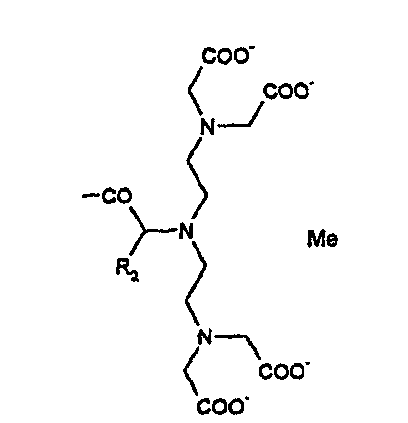 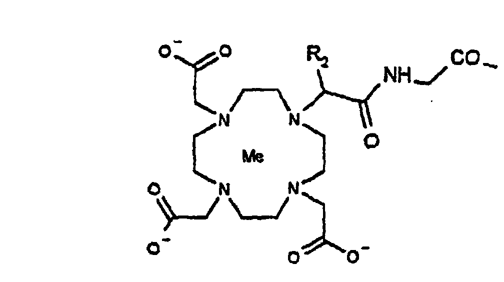 und 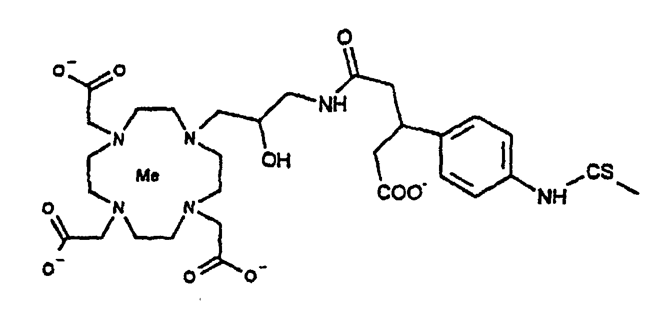
mit R₂ in der Bedeutung Wasserstoff, -(CH₂)₁₋₃-COOH, geradkettiges oder verzweigtes (C₁-C₄)-Alkyl, geradkettiges oder verzweigtes C₁-C₄-Hydroxyalkyl, Phenyl oder Benzyl, Me als Metallkation eines Elements der Ordnungszahl 21 bis 29, 39, 42, 44 oder 57-83 sowie gegebenenfalls Kationen anorganischer und/oder organischer Basen, Aminosäuren oder Aminosäurenamide oder
R₁ in der Bedeutung von 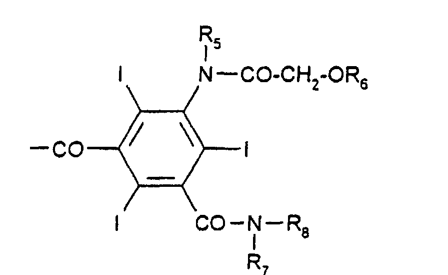 mit
R₅ in der Bedeutung Wasserstoff oder C₁-C₂-Alkyl oder einer -(CH₂)₁₋₃-COOH-Gruppe,
R₆ in der Bedeutung Wasserstoff oder Methyl und
R₇, R₈ gleich oder verschieden in der Bedeutung Wasserstoff oder geradkettiges Alkyl mit 2-6 C-Atomen oder verzweigtkettiges Alkyl mit 3-6 C-Atomen, wobei beide Alkylreste mit 1-5 OH Gruppen substituiert sein können,
R₃ für die Hydroxy- oder C₁-C₂-Alkoxy-Gruppe,
R₄ für die Hydroxy-, Methyl- oder Methoxygruppe,
Y die Reste ―(CH₂)ₚ[O-CH₂-CH₂]ₒ, 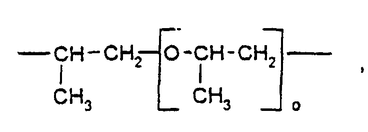 worin
o die Zahlen 10-200 und
p die Zahlen 2-20 bedeuten
oder
Y den Rest -[CH₂]₁₀-CO-NH-[CH₂]₂- bedeutet, wobei im letzteren Fall R₄ für -N(R₁₀)-Z[N(R₁₀)₂]₂ und R₃ für den Rest -NH R₉ steht,
worin R₉ Wasserstoff, Benzyloxycarbonyl, 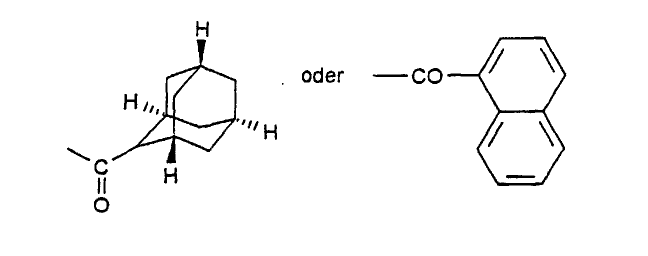
R₁₀ den Rest 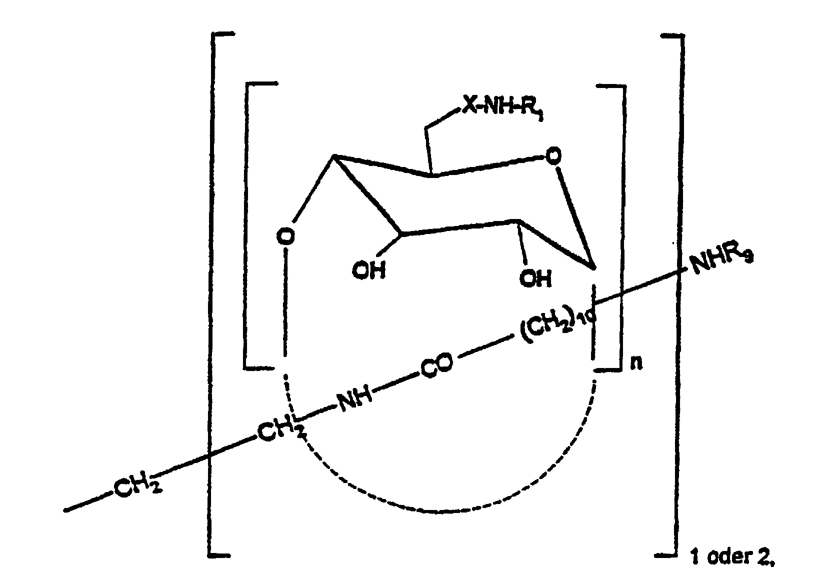 mit den oben angegebenen Bedeutungen für n, R₁ und R₉ und
Z den Rest 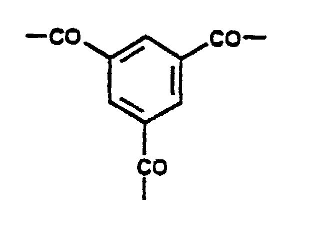 darstellen.

3. Pharmazeutische Mittel enthaltend mindestens einen Pseudopolyrotaxan-Komplex nach Anspruch 2, gegebenenfalls mit den in der Galenik üblichen Zusätzen.

4. Verwendung von mindestens einem Pseudopolyrotaxan-Komplex nach Anspruch 2 für die Herstellung von Mitteln für die NMR- oder Röntgendiagnostik.

5. Verwendung von mindestens einem Pseudopolyrotaxan-Komplex nach Anspruch 2 für die Herstellung von Mitteln für die Angiographie.

6. Verwendung von mindestens einem Pseudopolyrotaxan-Komplex nach Anspruch 2 für die Herstellung von Mitteln für die Lymphographie.

7. Verfahren zur Herstellung von Pseudopolyrotaxan-Komplexen gemäß Anspruch 2, **dadurch gekennzeichnet, daß** man Verbindungen der Formel II 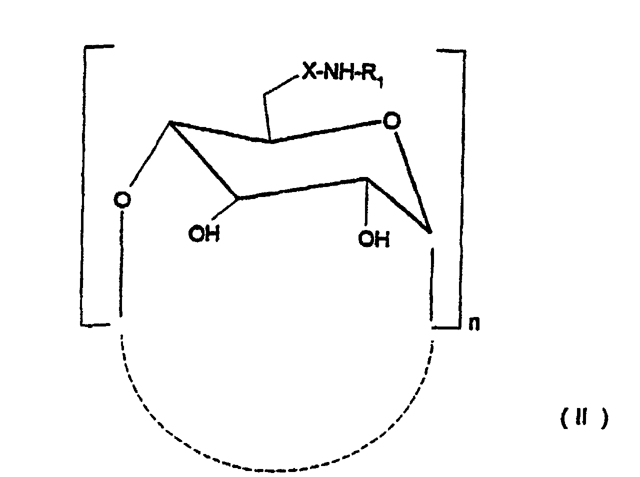 worin X, R₁ und n die oben angegebenen Bedeutungen haben, mit Verbindungen der Formel III
R₃―Y―R₁₁ (III),
worin
Y und R₃ die oben angegebenen Bedeutungen haben und R₁₁ Hydroxy, Methyl, Methoxy oder, falls Y für -(CH₂)₁₀-CO-NH (CH₂)₂ - steht,
den Rest -N(R₁₂)Z [N(R₁₂)₂]₂ bedeutet,
R₁₂ den Rest -(CH₂)₂-NH-CO-(CH₂)₁₀-NHR₉ mit R₉ in der oben angebenen Bedeutung darstellt, umsetzt.

## Claims

1. Pseudopolyrotaxanes that contain metal complex or iodine-containing benzene derivatives as imaging components for MRT diagnosis and diagnostic radiology.

2. Pseudopolyrotaxanes of formula I 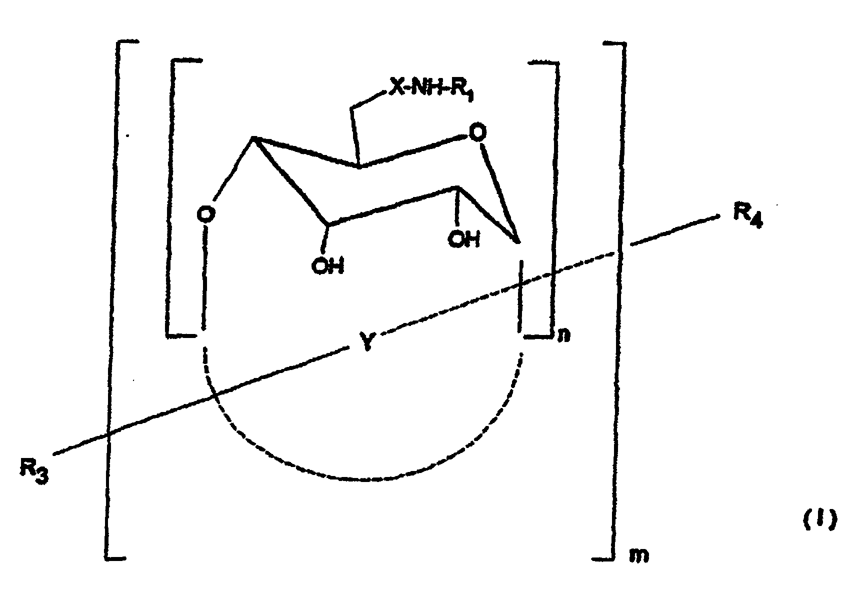 in which
n means the numbers 6, 7 or 8,
m means the numbers 2 to 50,
X means a direct bond or the radical -O-CO-CH(CH₃)-,
R₁ means the opacifying radicals 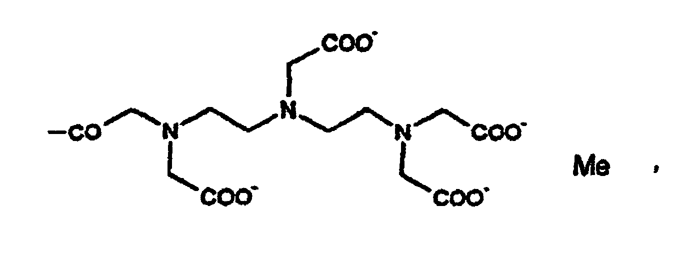 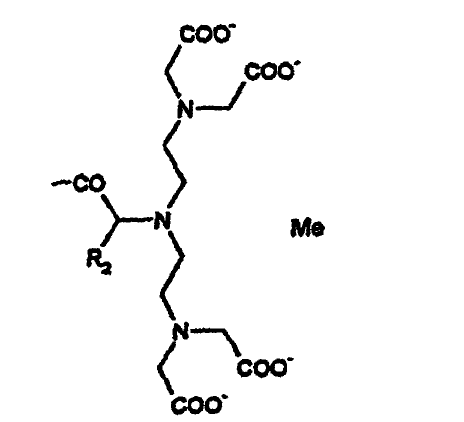 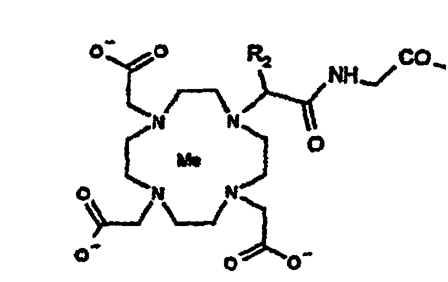 and 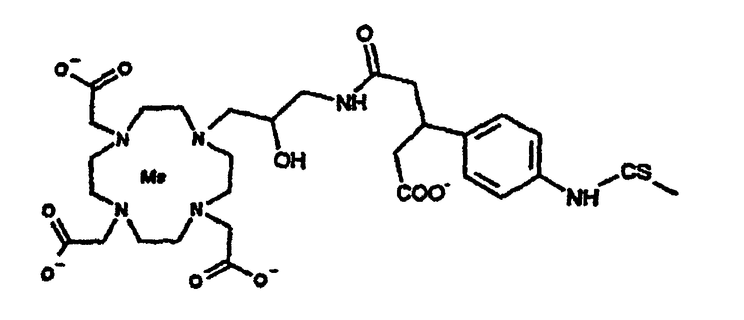
with R₂ meaning hydrogen, -(CH₂)₁₋₃-COOH, straight-chain or branched (C₁-C₄)-alkyl, straight-chain or branched (C₁-C₄)-hydroxyalkyl, phenyl or benzyl, Me as a metal cation of an element of atomic numbers 21 to 29, 39, 42, 44 or 57-83 as well as optionally cations of inorganic and/or organic bases, amino acids or amino acid amides or
R₁ meaning 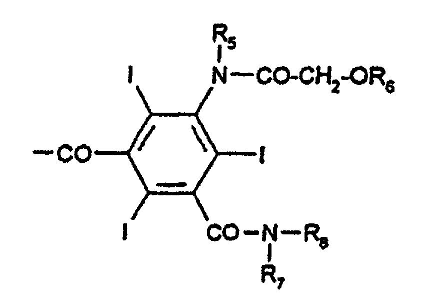
with R₅ meaning hydrogen or C₁-C₂-alkyl or a -(CH₂)1-3-COOH group,
R₆ meaning hydrogen or methyl, and
R₇, R₈, the same or different, meaning hydrogen or straight-chain alkyl with 2-6 C atoms or branched-chain alkyl with 3-6 C atoms, where both alkyl radicals can be substituted with 1-5 OH groups,
R₃ stands for a hydroxy or C₁-C₂-alkoxy group,
R₄ stands for a hydroxy, methyl or methoxy group,
Y means the radicals -(CH₂)ₚ[O-CH₂-CH₂]ₒ, 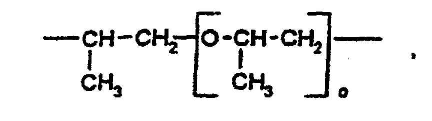 in which
o means the numbers 10-200, and
p means the numbers 2-20 or
Y means the radical -[CH₂]₁₀-CO-NH-[CH₂]₂-, where, in the latter case, R₄ stands for -N(R₁₀)-Z[N(R₁₀)₂]₂ and R₃ stands for the radical -NHR₉, in which R₉ represents hydrogen, benzyloxycarbonyl, 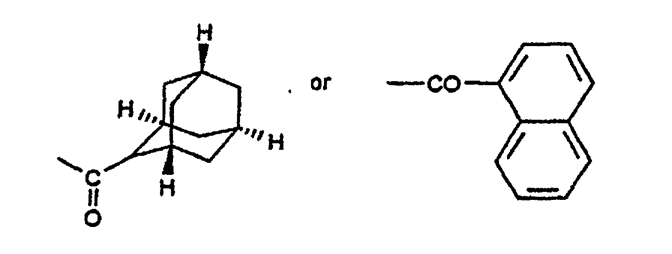
R₁₀ represents the radical 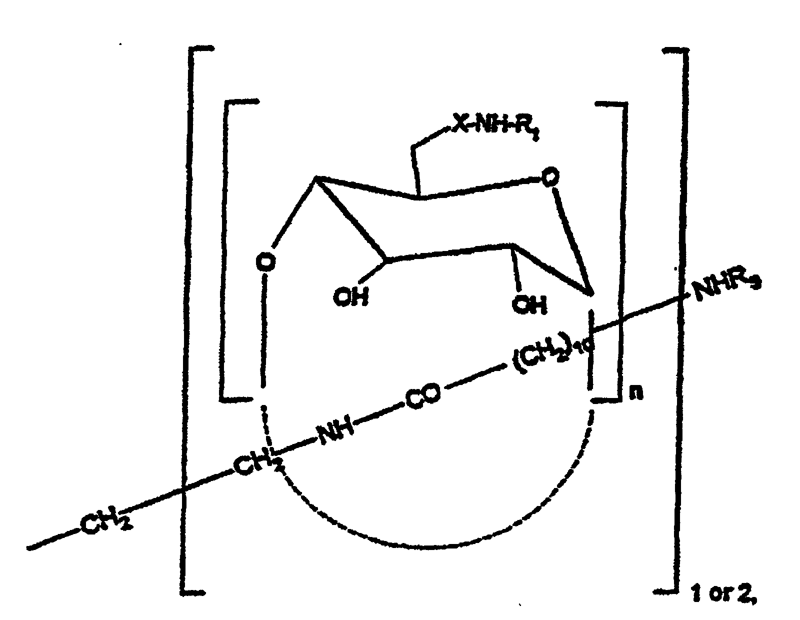 with the above-indicated meanings for n, R₁ and R₉, and Z represents the radical

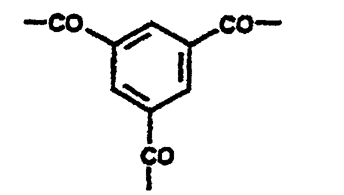

3. Pharmaceutical agents that contain at least one pseudopolyrotaxane complex according to Claim 2, optionally with the additives that are commonly used in pharmaceutical technology.

4. Use of at least one pseudopolyrotaxane complex according to Claim 2 for the production of agents for NMR diagnosis or diagnostic radiology.

5. Use of at least one pseudopolyrotaxane complex according to Claim 2 for the production of agents for angiography.

6. Use of at least one pseudopolyrotaxane complex according to Claim 2 for the production of agents for lymphography.

7. Process for the production of pseudopolyrotaxane complexes according to Claim 2, **characterized in that** compounds of formula II 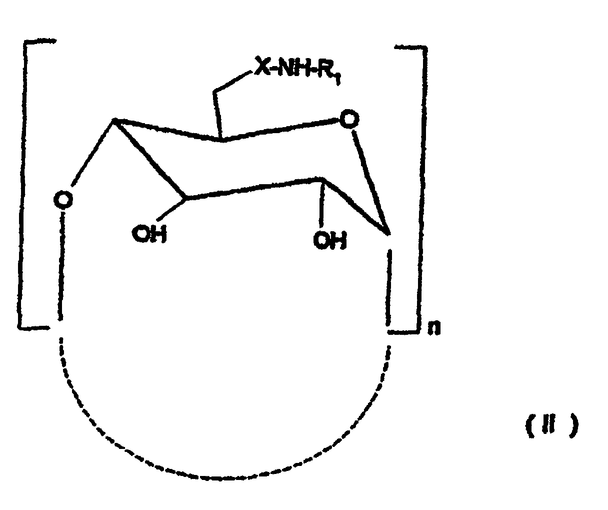 in which X, R₁ and n have the above-indicated meanings, are reacted with compounds of formula III
R₃-Y-R₁₁ (III),
in which
Y and R₃ have the above-indicated meanings, and R₁₁ means hydroxyl, methyl, methoxy or, if Y stands for -(CH₂)₁₀-CO-NH(CH₂)₂, the radical -N(R₁₂)Z[N(R₁₂)₂]₂,
R₁₂ represents the radical -(CH₂)₂-NH-CO-(CH₂)₁₀-NHR₉ with R₉ having the above-indicated meaning.

## Revendications

1. Pseudopolyrotaxanes contenant en tant que composant d'imagerie pour diagnostic par RMN et aux rayons X un complexe métallique ou des dérivés benzéniques iodés.

2. Pseudopolyrotaxanes de formule I 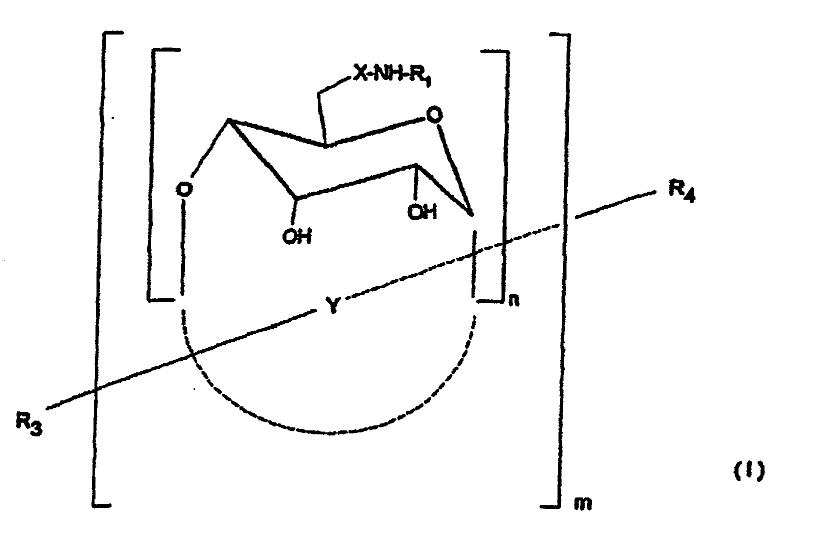 dans laquelle
n représente les nombres 6, 7 ou 8,
m représente les nombres 2 à 50,
X représente une liaison directe ou le radical -O-CO-CH(CH₃)-,
R₁ représente les radicaux générateurs de contraste 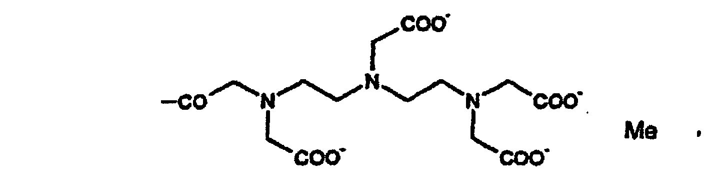 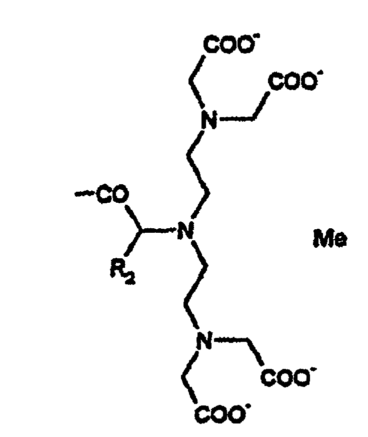 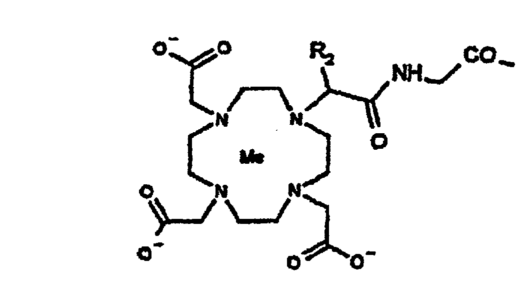 et 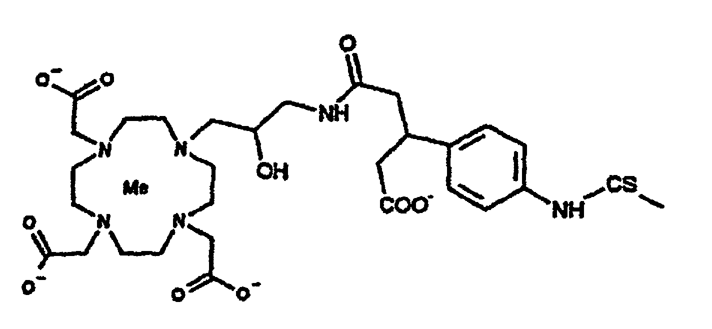
où R₂ représente un atome d'hydrogène, un groupe -(CH₂)₁₋₃-COOH, un groupe alkyle en C₁-C₄ à chaîne droite ou ramifiée, un groupe hydroxyalkyle en C₁-C₄ à chaîne droite ou ramifiée, le groupe phényle ou benzyle,
Me représente un cation métallique d'un élément de nombre atomique 21 à 29, 39, 42, 44 ou 57-83, ainsi qu'éventuellement des cations de bases minérales et/ou organiques, d'aminoacides, ou d'amides d'aminoacides, ou
R₁ représente 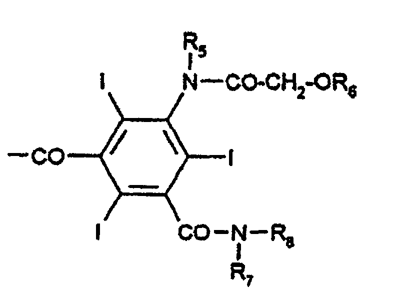 où
R₅ représente un atome d'hydrogène ou un groupe alkyle en C₁-C₂ ou un groupe -(CH₂)₁₋₃-COOH,
R₆ représente un atome d'hydrogène ou le groupe méthyle et
R₇, R₈ sont identiques ou différents et représentent un atome d'hydrogène ou un groupe alkyle à chaîne droite ayant de 2 à 6 atomes de carbone, ou un groupe alkyle à chaîne ramifiée ayant de 3 à 6 atomes de carbone, les deux radicaux alkyle pouvant être substitués par 1-5 groupes OH,
R₃ représente le groupe hydroxy ou un groupe alcoxy en C₁-C₂,
R₄ représente le groupe hydroxy, méthyle ou méthoxy,
Y représente les radicaux-(CH₂)ₚ[O-CH₂-CH₂]ₒ, 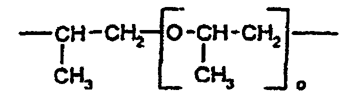 dans lesquels
o représente les nombres 10-200 et
p représente les nombres 2-20
ou
Y représente le radical-[CH₂]₁₀-CO-NH-[CH₂]₂-, dans ce dernier cas
R₄ représente le groupe -N(R₁₀)-Z[N(R₁₀)₂]₂ et R₃ représente le radical -NHR₉,
R₉ étant un atome d'hydrogène, le groupe benzyloxycarbonyle, 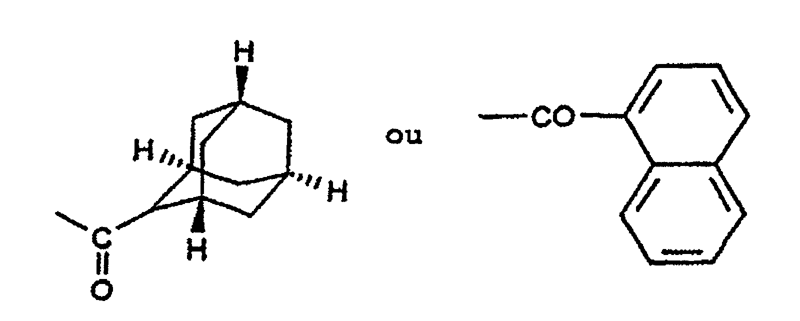
R₁₀ représente le radical 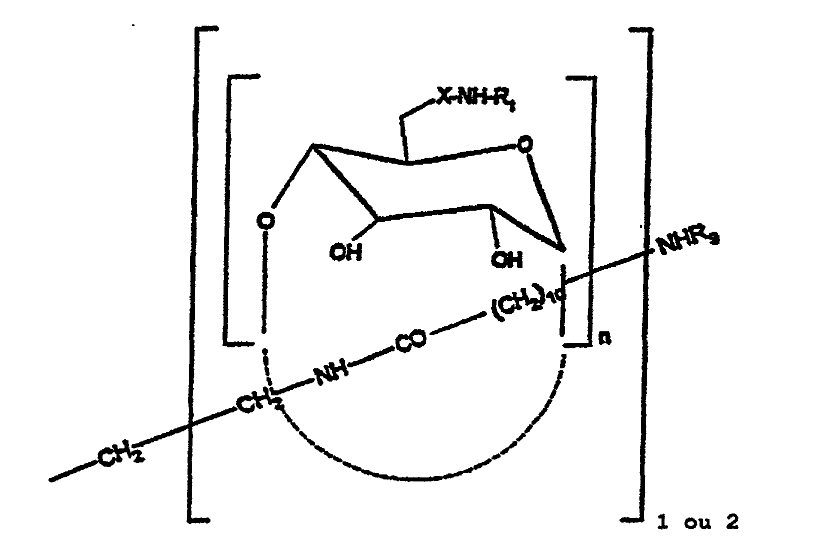 avec les significations données plus haut pour n, R₁ et R₉ et
Z représente le radical

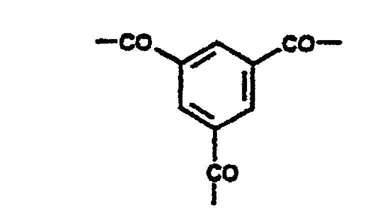

3. Produits pharmaceutiques contenant au moins un complexe de pseudopolyrotaxane selon la revendication 2, éventuellement avec les additifs usuels en formulation.

4. Utilisation d'au moins un complexe de pseudopolyrotaxane selon la revendication 2, pour la préparation de produits pour le diagnostic par RMN ou aux rayons X.

5. Utilisation d'au moins un complexe de pseudopolyrotaxane selon la revendication 2, pour la préparation de produits pour l'angiographie.

6. Utilisation d'au moins un complexe de pseudopolyrotaxane selon la revendication 2, pour la préparation de produits pour la lymphographie.

7. Procédé pour la préparation de complexes de pseudopolyrotaxanes selon la revendication 2, **caractérisé en ce qu'**on fait réagir des composés de formule II 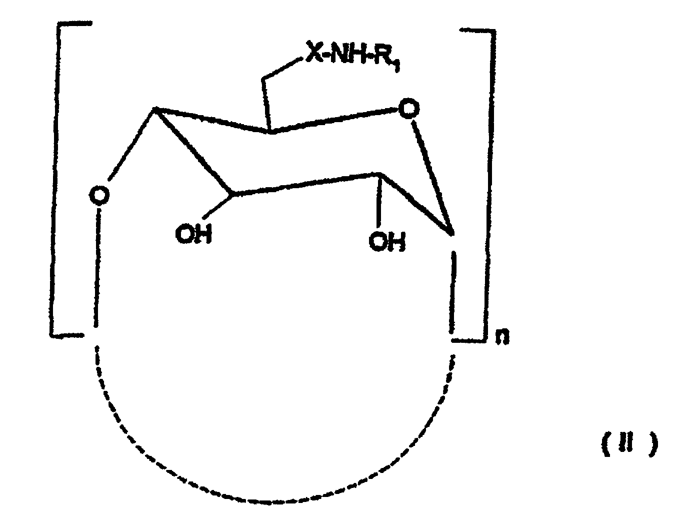 dans laquelle X, R₁ et n ont les significations données plus haut, avec des composés de formule III
R₃-Y-R₁₁ (III),
dans laquelle
Y et R₃ ont les significations données plus haut et R₁₁ représente le groupe hydroxy, méthyle, méthoxy ou, si Y représente-(CH₂)₁₀-CO-NH(CH₂)₂-, R₁₁ représente le radical -N(R₁₂)Z[N(R₁₂)₂]₂,
R₁₂ représentant le radical-(CH₂)₂-NH-CO-(CH₂)₁₀-NHR₉, R₉ ayant la signification donnée plus haut.
